# EUROPEAN PATENT SPECIFICATION

(11) **EP 1 945 607 B1**
(45) Date of publication and mention of the grant of the patent: **14.10.2009**
(21) Application number: 05826478.9
(22) Date of filing: 09.11.2005
(51) Int. Cl.: C07C 253/30, C07C 255/41

(54) **A PROCESS FOR THE PREPARATION OF HIGHLY PURE (E) N,N-DIETHYL-2-CYANO-3-(3,4-DIHYDROXY- 5-NITRO PHENYL) ACRYLAMIDE (ENTACAPONE)**
VERFAHREN ZUR HERSTELLUNG VON HOCHREINEM (E)-N,N-DIETHYL-2-CYANO-3-(3,4-DIHYDRO-5-NITROPHENYL)ACRYLAMID (ENTACAPON)
PROCÉDÉ POUR LA PRÉPARATION DE (E)-N,N-DIÉTHYL-2-CYANO-3-(3,4-DIHYDROXY-5-NITROPHÉNYL)ACRYLAMIDE (ENTACAPONE) EXTRÊMEMENT PUR

(43) Date of publication of application: 23.07.2008
(73) Proprietor: USV Limited, Mumbai 400088, Maharashtra (IN)
(72) Inventor: TARUR, Venkatasubramanian, Radhakrishnan, Mumbai 400 080 (IN); SATHE, Dhananjay, Govind, Thane 400 602 Maharashtra (IN); BHISE, Nandu, Baban, Mumbai 400 080 Maharashtra (IN); NAIDU, Avinash, Venkatraman, Thane 421 202 Maharashtra (IN); AHER, Umesh, Parashram, Kalyan 421 301 Maharashtra (IN); PATIL, Sachin, Shivaji, Kaylan 427 304 Maharashtra (IN)
(74) Representative: Schön, Christoph
(86) International application number: PCT/IN2005/000363
(87) International publication number: WO 2007/054950

(56) References cited:
- WO-A-20/05066117
- WO-A-20/05070881
- GB-A- 2 200 109
- US-A- 5 135 950

## Description

### Technical Field

The invention relates to a process for the preparation of highly pure (E) N,N-diethyl-2-cyano-3-(3,4-dihydroxy-5-nitro phenyl) acrylamide (Entacapone) of the formula I:

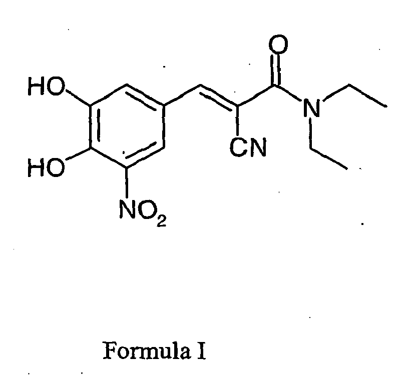

### Background and Prior Art

US 4,963, 590 describes catechol derivatives including N, N-diethyl-2-cyano-3-(3,4-dihydroxy-5-nitro phenyl) acrylamide as being useful in the treatment of diseases like central or peripheral nervous system disorders, Parkinson's disease or parkinsonian disorders. Process for the preparation of N, N-diethyl-2-cyano-3-(3,4-dihydroxy-5-nitro phenyl) acrylamide comprises condensation of 3,4-dihydroxy-5-nitro benzaldehyde and N,N-hydrochloric diethyl cyanoacetamide in the presence of piperidine-acetate in dry ethanol (Example 100). The condensation reaction and yield are reported to be very long (overnight) and 73%, respectively. The process is not, therefore, commercially viable for industrial scale production of entacapone and economical.

US 5,135,950 reports that N,N-diethyl-2-cyano-3-(3,4-dihydroxy-5-nitrophenyl)acrylamide is a mixture of two geometric isomers namely E- and Z-isomers (70 - 80% E-isomer and 30-20% Z-isomer) of the following formulae I and II, respectively:

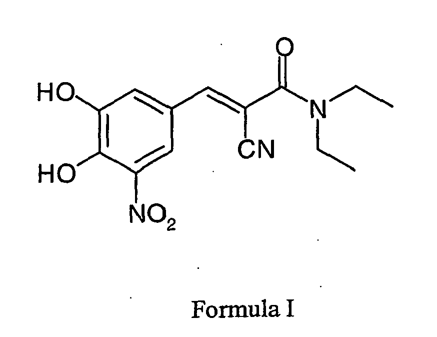

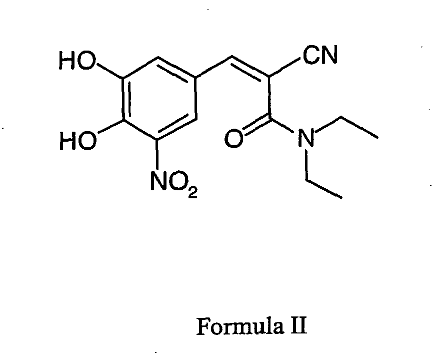

This patent relates to a stable and crystallographically essentially pure polymorphic form A of E- isomer of N,N-diethyl-2-cyano-3-(3,4-dihydroxy-5-nitrophenyl)acrylamide obtained by purifying the crude product by recrystallisation from lower aliphatic carboxylic acid such as formic acid or acetic acid with catalytic amount of hydrochloric or hydrobromic acid. The purification procedure increases the process duration and renders it commercially unviable for industrial scale production.

WO 2005/063693 describes a process for the preparation of entacapone using 3-alkoxy-4-hydroxy-5-nitrobenzaldehyde.

WO 2005/070881 describes a process for the production of (E)-entacapone polymorphic form A. 3,4Dihydroxy-5-nitrobenzaldehyde is condensed with N,N-diethylcyanoacetamide in the presence of a base like piperidine in alcohol like isopropanol. The reaction mixture is poured into a mixture of aqueous ethyl acetate and water followed by pH adjustment with acetic acid. The product is separated from the organic layer to yield (E) isomer of entacapone having 99.7% purity. The condensation reaction itself is reported to be quite long (10 - 15 hours).

Novel polymorphic forms C and D of entacapone are disclosed in WO2005/066117. 3,4Dihydroxy-5-nitrobenzaldehyde is condensed with N,N-diethylcyanoacetamide in the presence of a base like piperidine in isopropanol followed by addition of acetic acid and crystallization with a mixture of alcohols to obtain polymorphic form C. Polymorphic form D is obtained from form C or form A by dissolution in solvents such as polar organic solvents aliphatic alcohols or sulfoxides.

WO 2005/063695 and WO 2005/063696 also disclose novel polymorphs C, D and E of Entacapone by crystallisation.

### Objects of the Invention

An object of the invention is to provide a process for the preparation of (E)-N, N-diethyl-2-cyano-3-(3,4-dihydroxy-5-nitrophenyl) acrylamide, which gives the product in high purity and good yield.

Another object of the invention is to provide a process for the preparation of (E)-N, N-diethyl-2-cyano-3-(3,4-dihydroxy-5-nitrophenyl) acrylamide, which reduces the process duration and is simple, easy and convenient to carry out and is commercially viable and economical.

Another object of the invention is to provide (E)-N, N-diethyl-2-cyano-3-(3,4-dihydroxy-5-nitrophenyl) acrylamide in very high purity and good yield.

### Detailed Description of invention

According to the invention there is provided a process for the preparation of highly pure (E)-N,N-diethyl-2-cyano-3-(3,4-dihydroxy-5-nitrophenyl) acrylamide (Entacapone) of the formula I 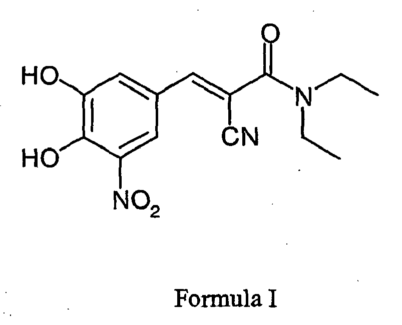 comprising
i) condensing 3,4-dihydroxy-5-nitro benzaldehyde with N,N-diethyl cyano acetamide in an organic solvent in the presence of an organic base and an acid under reflux conditions followed by distilling off the solvent under vacuum and treating the residue with a lower aliphatic carboxylic acid at 40 - 80°C;
ii) extracting the residue with a chlorinated solvent and distilling off the solvent under vacuum;
iii) extracting the residue with an organic solvent to obtain crude N,N-diethyl-2-cyano-3-3,4-dihydroxy-5-nitrophenyl)acrylamide; and
iv) treating the crude product N,N-diethyl-2-cyano-3-(3,4-dihydroxy-5-nitrophenyl) acrylamide with a mixture of organic alcohol and organic acid in the molar ratio of the crude product to organic alcohol 1:5 to 15 and crude product to organic acid 1:1 to 3 under reflux conditions.

The organic solvent used in step (i) is selected from lower aliphatic alcohol like methanol, ethanol, n-propanol, isopropanol or mixtures thereof or is preferably ethanol. The organic base used in step (i) is selected from piperidine morpholine, N-methyl morpholine or pyrrolidine or is preferably piperidine. The acid used in step (i) is organic acid selected from lower aliphatic carboxylic acid or inorganic acid selected from sulfuric acid or hydrochloric acid. The lower aliphatic carboxylic acid used in step (i) is preferably acetic acid or formic acid. Preferably, the residue in step (i) is treated at 65°C.

The chlorinated solvent used in step (ii) is selected from methylene chloride or chloroform or is preferably methylene chloride.

The organic solvent used in step (iii) is selected from polar and non-polar solvents such as alcohols, ketones, esters or mixtures thereof. The organic solvent is preferably ester selected from methyl acetate, ethyl acetate, propyl acetate or mixtures thereof or is preferably ethyl acetate.

The mixture of organic alcohol and organic,acid used in step (iv) is selected from aliphatic alcohol like methanol, ethanol, n-propanol or isopropanol and lower aliphatic carboxylic acid like acetic acid, formic acid, methane sulfonic acid or trifluoro acetic acid or is preferably, methanol and acetic acid.

Preferably, the molar ratio of the crude product to organic alcohol is 1: 8 and the crude product to organic acid is 1:2.

The product namely N,N-diethyl-2-cyano-3-(3,4-dihydroxy-5-nitrophenyl) acrylamide is recovered from the reaction mixture of step (iv) by filtering the reaction mixture, precipitating the product from the filtrate by gradually cooling the filtrate at 0-30°C preferably 10 - 50° C and drying the precipitate at 60 - 70° C preferably in an electric oven.

The process of the invention affords (E)-N,N-diethyl-2-cyano-3-(3,4-dihydroxy-5-nitrophenyl) acrylamide in high purity ( > 99.8%) and in good yield (about 53.68%) with Z- isomer content < 0.5%. The process duration is reduced and the process is simple, easy and convenient to carry out and is commercially viable and economical.

The following experimental example is illustrative of the invention but not limitative of the scope thereof:

### Example 1

A mixture of 3,4-dihydroxy-5-nitro benzaldehyde (500g), N,N-diethyl cyano acetamide (575ml), acetic acid (375ml) and piperidine (500ml) in ethanol (4.51) were refluxed for 6 hrs. Ethanol was distilled off under vacuum and 1.5 1 of formic acid was added to the residue at 65° C and stirred for 30 mins at 65° C. The reaction mixture was cooled to R T and charged with methylene dichloride. The organic layer was separated and washed twice with 2 x 3.5 1 water. Methylene dichloride was distilled off under vacuum from the organic layer and the residue was treated with ethyl acetate 1.25 1. The yellow solid was filtered out from the solvent, washed with 1.25 1 ethyl acetate and dried in an electric oven at 60-70° C to obtain crude N,N-diethyl-2-cyano-3-(3,4-dihydroxy-5-nitro phenyl) acrylamide.
Yield of crude entacapone 495g (59.63%), Purity 99.2++
Z- isomer 0.5%

The crude product (495 g), methanol (3960ml) and acetic acid (990ml) were refluxed for 1hr. The clear solution was filtered and the product was precipitated by gradually cooling the filtrate to 10-15° C. The solid was filtered out, washed twice with cold ethyl acetate (2 x 990 ml) at 15°C and dried in an electric oven at 60-70° C.
Yield of entacapone based on the crude product 445.5g (90 %). Purity 99.8%.
Z-isomer 0.02 %

## Claims

1. A process for the preparation of highly pure (E)-N,N-diethyl-2-cyano-3-(3,4-dihydroxy-5-nitrophenyl)acrylamide (Entacapone) of the formula I 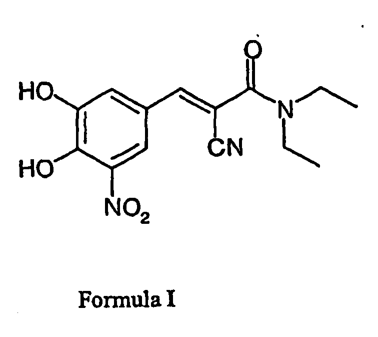 comprising
i) condensing 3,4-dihydroxy-5-nitro benzaldehyde with N,N-diethyl cyano acetamide in an organic solvent in the presence of an organic base and an acid under reflux conditions followed by distilling off the solvent under vacuum and treating the residue with a lower aliphatic carboxylic acid at 40 - 80°C;
ii) extracting the residue with a chlorinated solvent and distilling off the solvent under vacuum;
iii) extracting the residue with an organic solvent to obtain crude N,N-diethyl-2-cyano-3-(3,4-dihydroxy-5-nitrophenyl)acrylamide; and
iv) treating the crude product N,N-diethyl-2-cyano-3-(3,4-dihydroxy-5-nitrophenyl) acrylamide with a mixture of organic alcohol and organic acid in the molar ratio of the crude product to organic alcohol 1:5 to 15 and crude product to organic acid 1:1 to 3 under reflux conditions.

2. The process as claimed in claim 1, wherein the organic solvent used in step (i) is selected from lower aliphatic alcohol like methanol, ethanol, n-propanol, iso-propanol or mixtures thereof or is preferably ethanol.

3. The process as claimed in claim 1, wherein organic base used in step (i) is selected from piperidine, morpholine, N-methyl morpholine or pyrrolidine or is preferably piperidine and the acid used in step (i) is selected from lower aliphatic carboxylic acid like acetic acid or formic acid or inorganic acid selected from hydrochloric acid or sulphuric acid or is preferably acetic acid or formic acid.

4. The process as claimed in claim 1, wherein the residue in step (i) is treated at 65°C.

5. The process as claimed in claim 1, wherein the chlorinated solvent used in step (ii) is selected from chlorofom or methylene dichloride or is preferably methylene dichloride.

6. The process as claimed in claim 1, wherein the organic solvent used in step (iii) is selected from polar and non-polar solvents such as alcohols, ketones or esters or mixtures thereof or is preferably ester, preferably ethyl acetate.

7. The process as claimed in claim 1 wherein the mixture of organic alcohol and organic acid used in step (iv) is selected from aliphatic alcohol like methanol, ethanol, n-propanol or iso-propanol and lower aliphatic carboxylic acid like acetic acid, formic acid or trifluoro acetic acid or is preferably methanol and acetic acid.

8. The process as claimed in claim 1, wherein molar ratio of the crude product to alcohol is 1:8 and the crude product to organic acid is 1: 2.

9. The process as claimed in any one of claims 1 to 8, wherein (E)-N,N-diethyl-2-cyano-3-(3,4-dihydroxy-5-nitrophenyl)acrylamide of the formula I 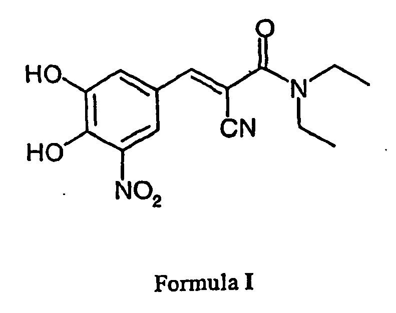 is obtained in high purity of greater than 99.8%.

## Patentansprüche

1. Verfahren zur Herstellung eines hochreinen (E)-N,N-Diethyl-2-cyano-3-(3,4-dihydroxy-5-nitrophenyl)-acrylamids (Entacapon) der Formel I 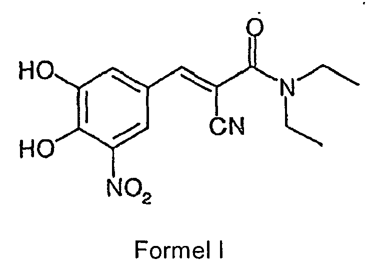 wobei das Verfahren die folgenden Stufen umfasst:
i) Kondensieren von 3,4-Dihydroxy-5-nitrobenzaldehyd mit N,N-Diethylcyanoacetamid in einem organischen Lösemittel in Gegenwart einer organischen Base und einer Säure unter Rückflussbedingungen, gefolgt von einem Abdestillieren des Lösemittels unter Vakuum und Behandeln des Rückstands mit einer niedrigen aliphatischen Carbonsäure bei 40 - 80 °C;
ii) Extrahieren des Rückstandes mit einem chlorierten Lösemittel und Abdestillieren des Lösemittels unter Vakuum;
iii) Extrahieren des Rückstands mit einem organischen Lösemittel, um rohes N,N-Diethyl-2-cyano-3-(3,4-dihydroxy-5-nitrophenyl)-acrylamid zu erhalten; und
iv) Behandeln des rohen Produkts von N,N-Diethyl-2-cyano-3-(3,4-dihydroxy-5-nitrophenyl)-acrylamid mit einem Gemisch eines organischen Alkohols mit einer organischen Säure in einem Molverhältnis rohes Produkt : organischer Alkohol = 1 : 5 bis 15 und rohes Produkt : organische Säure = 1 : 1 bis 3 unter Rückflussbedingungen.

2. Verfahren nach Anspruch 1, worin das in Stufe (i) verwendete organische Lösemittel aus einem niedrigen aliphatischen Alkohol, wie Methanol, Ethanol, n-Propanol, Isopropanol oder Gemischen hiervon, ausgewählt ist oder vorzugsweise Ethanol ist.

3. Verfahren nach Anspruch 1, worin die in Stufe (i) verwendete organische Base aus Piperidin, Morpholin, N-Methylmorpholin oder Pyrrolidin ausgewählt ist oder vorzugsweise Piperidin ist und die in Stufe (i) verwendete Säure aus einer niedrigen aliphatischen Carbonsäure, wie Essigsäure oder Ameisensäure, oder einer aus Salzsäure oder Schwefelsäure ausgewählten anorganischen Säure ausgewählt ist oder vorzugsweise Essigsäure oder Ameisensäure ist.

4. Verfahren nach Anspruch 1, worin der Rückstand in Stufe (i) bei 65 °C behandelt wird.

5. Verfahren nach Anspruch 1, worin das in Stufe (ii) verwendete chlorierte Lösemittel aus Chloroform oder Methylendichlorid ausgewählt ist oder vorzugsweise Methylendichlorid ist.

6. Verfahren nach Anspruch 1, worin das in Stufe (iii) verwendete organische Lösemittel aus polaren und nicht-polaren Lösemitteln, wie Alkoholen, Ketonen oder Estern oder Gemischen hiervon, ausgewählt ist oder vorzugsweise ein Ester, vorzugsweise Ethylacetat, ist.

7. Verfahren nach Anspruch 1, worin das in Stufe (iv) verwendete Gemisch aus organischem Alkohol und organischer Säure aus einem aliphatischen Alkohol, wie Methanol, Ethanol, n-Propanol oder Isopropanol, und einer niedrigen aliphatischen Carbonsäure, wie Essigsäure, Ameisensäure oder Trifluoressigsäure, ausgewählt ist oder vorzugsweise Methanol und Essigsäure ist.

8. Verfahren nach Anspruch 1, worin das Molverhältnis rohes Produkt : Alkohol 1 : 8 beträgt und das Molverhältnis rohes Produkt : organische Säure 1 : 2 beträgt.

9. Verfahren nach einem der Ansprüche 1 bis 8, worin (E)-N,N-Diethyl-2-cyano-3-(3,4-dihydroxy-5-nitrophenyl)-acrylamid der Formel I 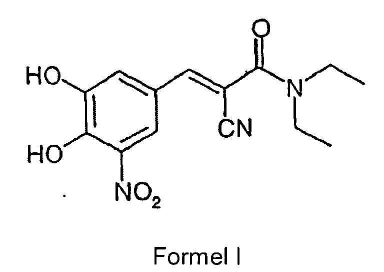 in einer hohen Reinheit von größer als 99,8 % erhalten wird.

## Revendications

1. Procédé pour la préparation de (E)-N,N-diéthyl-2-cyano-3-(3,4-dihydroxy-5-nitrophényl)acrylamide (Entacapone) hautement pure de formule I 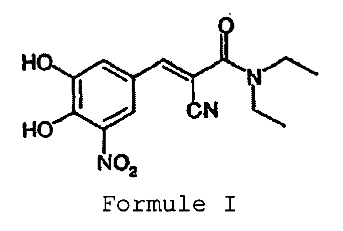 comprenant
i) la condensation du 3,4-dihydroxy-5-nitrobenzaldéhyde avec du N,N-diéthylcyanoacétamide dans un solvant organique en présence d'une base organique et d'un acide sous reflux, suivi par la distillation du solvant sous vide et par le traitement du résidu avec un acide carboxylique aliphatique inférieur à 40-80 °C ;
ii) l'extraction du résidu avec un solvant chloré et la distillation du solvant sous vide ;
iii) l'extraction du résidu avec un solvant organique pour obtenir du N,N-diéthyl-2-cyano-3-(3,4-dihydroxy-5-nitro-phényl)acrylamide brut ; et
iv) le traitement du N,N-diéthyl-2-cyano-3-(3,4-dihydroxy-5-nitrophényl)acrylamide sous forme de produit brut avec un mélange d'alcool organique et d'acide organique en un rapport molaire du produit brut à l'alcool organique de 1:5 à 15 et du produit brut à l'acide organique de 1:1 à 3 sous reflux.

2. Procédé selon la revendication 1, dans lequel le solvant organique utilisé dans l'étape (i) est choisi parmi un alcool aliphatique inférieur comme le méthanol, l'éthanol, le n-propanol, l'isopropanol ou des mélanges de ceux-ci ou est de préférence l'éthanol.

3. Procédé selon la revendication 1, dans lequel la base organique utilisée dans l'étape (i) est choisie parmi la pipéridine, la morpholine, la N-méthylmorpholine ou la pyrrolidine ou est de préférence la pipéridine et l'acide utilisé dans l'étape (i) est choisi parmi un acide carboxylique aliphatique inférieur comme l'acide acétique ou l'acide formique ou un acide inorganique choisi parmi l'acide chlorhydrique ou l'acide sulfurique ou est de préférence l'acide acétique ou l'acide formique.

4. Procédé selon la revendication 1, dans lequel le résidu de l'étape (i) est traité à 65 °C.

5. Procédé selon la revendication 1, dans lequel le solvant chloré utilisé dans l'étape (ii) est choisi parmi le chloroforme ou du dichlorure de méthylène ou est de préférence du dichlorure de méthylène.

6. Procédé selon la revendication 1, dans lequel le solvant organique utilisé dans l'étape (iii) est choisi parmi des solvants polaires ou non polaires tels que des alcools, des cétones ou des esters ou des mélanges de ceux-ci ou est de préférence un ester, de préférence de l'acétate d'éthyle.

7. Procédé selon la revendication 1, dans lequel le mélange d'alcool organique et d'acide organique utilisé dans l'étape (iv) est choisi parmi un alcool aliphatique comme le méthanol, l'éthanol, le n-propanol ou l'isopropanol et un acide carboxylique aliphatique inférieur comme l'acide acétique, l'acide formique ou l'acide trifluoroacétique ou est de préférence le méthanol ou l'acide acétique.

8. Procédé selon la revendication 1, dans lequel le rapport molaire du produit brut à l'alcool est de 1:8 et du produit brut à l'acide organique est de 1:2.

9. Procédé selon l'une quelconque des revendications 1 à 8, dans lequel le (E)-N,N-diéthyl-2-cyano-3-(3,4-dihydroxy-5-nitrophényl)acrylamide de formule I 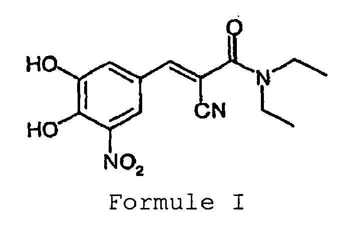 est obtenu avec une pureté élevée supérieure à 99,8 %.
